# EUROPEAN PATENT SPECIFICATION

(11) **EP 3 875 154 B1**
(45) Date of publication and mention of the grant of the patent: **21.05.2025**
(21) Application number: 19878780.6
(22) Date of filing: 31.10.2019
(51) Int. Cl.: A61Q 5/06, A61K 8/34, A61K 8/894, A61K 8/41

(54) **HAIR COSMETIC AND HAIR TREATMENT METHOD USING SAME**
HAARKOSMETIKUM UND HAARBEHANDLUNGSVERFAHREN MIT VERWENDUNG DAVON
PRODUIT COSMÉTIQUE CAPILLAIRE ET PROCÉDÉ DE TRAITEMENT DES CHEVEUX L'UTILISANT

(30) Priority: 02.11.2018 JP 2018206972
(43) Date of publication of application: 08.09.2021
(73) Proprietor: Shiseido Company, Ltd., Chuo-ku Tokyo 104-0061 (JP)
(72) Inventor: KOJIRI, Momoka, Tokyo 104-0061 (JP); KURASHIMA, Takumi, Tokyo 104-0061 (JP); YOSHIBA, Ryo, Tokyo 104-0061 (JP)
(74) Representative: Ter Meer Steinmeister & Partner
(86) International application number: PCT/JP2019/042715
(87) International publication number: WO 2020/090952

(56) References cited:
- EP-A1- 3 295 820
- JP-A- 2011 503 059
- JP-A- 2016 050 184
- JP-A- 2017 128 513
- DATABASE GNPD [online] MINTEL; 25 November 2016 (2016-11-25), ANONYMOUS: "Liquid Umbrella Waterproofing Strands", XP055960239, retrieved from https://www.gnpd.com/sinatra/recordpage/4436531/ Database accession no. 4436531
- DATABASE GNPD [online] MINTEL; 27 September 2017 (2017-09-27), ANONYMOUS: "Smooth Spray", XP055960242, retrieved from https://www.gnpd.com/sinatra/recordpage/5024181/ Database accession no. 5024181
- ANONYMOUS: "Marketing Bulletin SILSOFT CLX-E conditioning agent Speciality fluids -personal care", INTERNET CITATION, 1 October 2015 (2015-10-01), pages 1 - 12, XP002788416, Retrieved from the Internet <URL:www.momentive.com>
- DATABASE GNPD [online] MINTEL; 13 August 2018 (2018-08-13), ANONYMOUS: "Easy Straight Effect Spray", XP055947001, retrieved from https://www.gnpd.com/sinatra/recordpage/5884817/ Database accession no. 5884817
- ANONYMOUS: "Marketing Bulletin SILSOFT CLX-E conditioning agent Speciality fluids -personal care ", 1 October 2015 (2015-10-01), pages 1 - 12, XP002788416, Retrieved from the Internet <URL:www.momentive.com>

## Description

### Technical Field

The present invention relates to a hair cosmetic. More specifically, the present invention relates to a hair cosmetic used with spraying in the form of a mist, which is the hair cosmetic capable of imparting a desired shape, smoothness, water repellency and the like to hair and also of holding these effects for a long time with good stability and sense of use, and a method for using the same.

### Background Art

Various hair care components such as silicones are mixed in hair washing cosmetics such as shampoos and conditioners, and hair finishing cosmetics such as hair styling agents and hair treatments. These hair care components adhere to damaged parts of hair, repair, coat the hair and thereby improving easiness in combing.

However, polymers mixed in the conventional hair cosmetics as hair care components were capable of repairing easiness in combing hair immediately after used, but most of them were tend to be easily washed away by washing and therefore they were insufficient in holding the effects for a long time or and a texture upon use.

For example, in Patent Document 1 and Patent Document 2, a hair cosmetic in which (a) a block polymer having a specific structure, (b) water and/or a lower alcohol solvent, (c) a thickener, and (d) silicone are mixed is disclosed and considered to be good in a straightening effect immediately after applied to hair and also good in stability and sense of use. However, the block polymer mixed in such a hair cosmetic is a resin having the backbone of a polyvinyl structure, and thus there is room to resolve a stiff and coarse feel, a shaggy feel and stickiness. Further, such a block polymer tends to be easily washed away thereby posing a problem of causing insufficient holding property of the hair straightening effect.

Patent Document 3 discloses a nonaqueous type hair cosmetic in which (A) dimethiconol, (B) a block-copolymer type poly(oxyethylene·oxypropylene)·alkylene·methylpolysiloxane copolymer, (C) volatile silicone, and (D) a vegetable oil are mixed. This hair cosmetic is good in transparency and straightening hair with straightness holding property, protects hair from heat of a hair iron and good in natural glossiness and smoothness. However, this nonaqueous type hair cosmetic had a drawback of causing a sense of weight due to the oil mixed, being easily washed away and having insufficient holding property of hair straightening effect.

Patent Document 4 describes a hair treatment method which uses a composition in which a silicone polymer having an alkoxy-(aminomethyl)-silyl group a terminal is mixed. The silicone polymer used here is considered to hold a hair form for a long time and demonstrate good aesthetic characteristics. However, the composition used in this hair treatment is nonaqueous, with a mixing amount of water being limited to less than 5%, and desired effects cannot be obtained when 5% or more of water is present.

### Citation List

### Patent Document

Patent Document 1: JP-B 6032725
Patent Document 2: JP-B 6292706
Patent Document 3: JP-A 2007-51078
Patent Document 4: JP-A 2016-525534

### Summary of Invention

### Technical Problem

Thus, an object of the present invention is to provide an aqueous hair cosmetic usable by spraying in the form of a mist, the hair cosmetic being capable of repairing hydrophobicity, easiness in combing and smoothness of damaged hair by suitably carrying out heat treatment after application of the cosmetic to hair and having good effect-holding properties for retaining the effects for a long time.

### Solution to Problem

The present inventors have found that a hair cosmetic which has good effect-holding properties and demonstrates a high water-repellent effect and a smooth sense of use can be obtained when a copolymer including a main chain having a polysiloxane structure and a polyoxyalkylene structure and a side chain having a trialkoxysilane group or a silanol group is used in combination with a cationic surfactant, whereby the present invention was accomplished.

That is, the present invention provides a hair cosmetic comprising:
(A) a copolymer including a main chain having a polysiloxane structure and a polyoxyalkylene structure and a side chain having a trialkoxysilane group or a silanol group;
(B) a cationic surfactant;
(C) a lower alcohol; and
(D) water.

### Advantageous Effects of Invention

The hair cosmetic according to the present invention is obtained as an aqueous hair cosmetic by using a specific copolymer including a main chain having a polysiloxane structure and a polyoxyalkylene structure and a side chain having a trialkoxysilane group or a silanol group in combination with a cationic surfactant, whereby the cosmetic can be used by spraying in the form of a mist, hydrophobicity (water repellency) is imparted to the hair surface, fast-drying and a silky feel are achieved, hair is easily arranged, heat resistance, chemical resistance and contamination resistance (pollen and the like) are improved with a good sense of use, and these effects can be held for an extended period of time. Description of Embodiments

Hereinafter, each of the components mixed in the hair cosmetic of the present invention is described in detail.

### (A) Copolymer including a main chain having a polysiloxane structure and a polyoxyalkylene structure and a side chain having a trialkoxysilane group or a silanol group

The polymer component (component A) mixed in the hair cosmetic of the present invention is a copolymer including a main chain (backbone) having a polysiloxane structure and a polyoxyalkylene structure and a side chain having a trialkoxysilane group or a silanol group (hereinafter abbreviated as "crosslinked silicone POA copolymer").

The crosslinked silicone POA copolymer has good adhesiveness to hair due to the hydrophilicity of the polyoxyalkylene structure present in the main chain (backbone) and imparts hydrophobicity (water repellency) to the hair surface due to the polysiloxane structure present in the main chain (backbone), and has good effect-holding properties because the trialkoxysilane group or the silanol group present in the side chain crosslinks at a comparatively low temperature thereby forming a solid (or strong) coat.

The polysiloxane structure constituting the backbone of the crosslinked silicone POA copolymer of the present invention comprises polydialkylsiloxane, preferably polydimethylsiloxane, wherein a part of alkyl groups (preferably methyl groups) may be substituted with a phenyl group.

The polyoxyalkylene structure constituting the backbone of the crosslinked silicone POA copolymer of the present invention is preferably a structure including, as a repeating unit, at least one selected from the group consisting of an oxyethylene group (EO), an oxypropylene group (PO), and an oxybutylene group (BO).

The crosslinked silicone POA copolymer in the present invention preferably further includes a side chain comprising an organic group. Examples of the organic group to be the side chain include a hydrocarbon group (preferably an alkyl group such as a straight chain or branched chain alkyl group having about 1 to 30 carbon atoms, or a phenyl group) which can be optionally substituted with an amino group, a hydroxyl group, a carboxyl group, and the like. The hydrocarbon group preferably has an amino group, and a hydrogen atom of such an amino group can further be replaced with an alkyl group and the like.

The crosslinked silicone POA copolymer of the present invention preferably further includes a nitrogen atom in the backbone thereof, and the side chain preferably bonds to the nitrogen atom.

The crosslinked silicone POA copolymer is polysilicone-29 (INCI name). Polysilicone-29 is defined as a complex silicone compound obtained by the reaction between a glycidopropyl-terminated dimethyl siloxane polymer and PEG-13 diglycidyl ether, diethylaminopropylamine, and aminopropyltriisopropoxysilane.

A commercial product can be used as the crosslinked silicone POA copolymer in the present invention. For example, a copolymer contained in a hair conditioning agent under the product name "Silsoft CLX-E" sold by Momentive Performance Materials Inc. can be particularly preferably used. This copolymer is a compound belonging to "Polysilicone-29." Such a copolymer has a structure in which the backbone includes a polysiloxane structure, a polyoxyalkylene structure and a nitrogen atom, a side chain has a trialkoxysilane group and a side chain comprises an organic group, wherein the polysiloxane structure includes polydimethylsiloxane, the polyoxyalkylene structure includes polyoxyethylene and polyoxyisopropylene, and the side chains bond to the nitrogen atom of the backbone. "Silsoft CLX-E" is a product containing the copolymer, dipropylene glycol and water.

A mixing amount of the crosslinked silicone POA copolymer (component A) in the hair cosmetic of the present invention based on the total cosmetic amount ranges from 0.05 to 5.0 mass%, preferably from 0.1 to 4.0 mass%, and more preferably from 0.1 to 3.0 mass%. When a mixing amount of the crosslinked silicone POA copolymer is less than 0.05 mass%, intended effects cannot be obtained, whereas when the mixing amount is more than 5.0 mass%, a sense of use may be reduced.

### (B) Cationic surfactant

The cationic surfactant (component B) mixed in the hair cosmetic of the present invention is quaternary ammonium salts represented by the following formula (I) 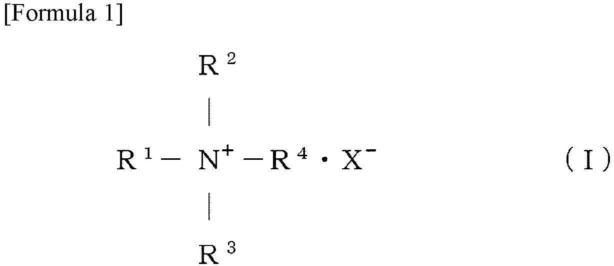 wherein R¹ is a straight chain or branched chain alkyl group having 6 to 35 carbon atoms, R², R³ and R⁴ may be the same or different and are a hydrogen atom or a straight chain or branched chain alkyl group having 1 to 3 carbon atoms, and X is a halogen ion or an organic anion.

More specifically, examples of R¹, the straight chain or branched chain alkyl group having 6 to 35 carbon atoms, include a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a ahenicosyl group, a docosyl group, a tricosyl group, a tetracosyl group, a pentacosyl group, a hexacosyl group, a heptacosyl group, an octacosyl group, nonacosyl group, a triacontyl group, a hentriacontyl group, dotriacontyl group, tritriacontyl group, tetratriacontyl group, a pentatriacontyl group, a myristoleyl group, a palmitoleyl group, an oleyl group, a linoyl group, a linoleyl group, a ricinoleyl group, and an isostearyl group. R², R³ and R⁴ may be the same or different as described above and are a hydrogen atom or a straight chain or branched chain alkyl group having 1 to 3 carbon atoms. Further, examples of X include halogen ions such as chlorine, iodine and bromine and organic anions such as methosulfate, ethosulfate, methophosphate and ethophosphate.

Examples of particularly preferable cationic surfactant in the present invention include behenyltrimethylammonium chloride (= behentrimonium chloride) wherein R¹ has 22 carbon atoms, R², R³ and R⁴ are all a methyl group, and X is chlorine, stearyltrimethylammonium chloride (= steartrimonium chloride) wherein R¹ has 18 carbon atoms, R², R³ and R⁴ are all a methyl group, and X is chlorine, and cetyltrimethylammonium chloride wherein R¹ has 16 carbon atoms, R², R³ and R⁴ are all a methyl group, and X is chlorine.

One or two or more of the above cationic surfactants can be mixed in combination in the hair cosmetic according to the present invention.

A mixing amount of (B) the cationic surfactant in the hair cosmetic of the present invention based on the total amount of the cosmetic is 0.01 to 1.0 mass%, preferably 0.03 to 0.5 mass%, and more preferably 0.05 to 0.5 mass%. When a mixing amount is less than 0.01 mass%, the stability of formulation is reduced, whereas when more than 1.0 mass% is mixed, a feel of use may become poor.

In the hair cosmetic of the present invention, a ratio (mass ratio) of a mixing amount of (A) the crosslinked silicone POA copolymer to a mixing amount of (B) the cationic surfactant, [(A)/(B)], is 0.5 or more, and preferably 0.8 or more. When this ratio is less than 0.5, sufficient effects may not be obtained.

The upper limit value of mixing amount ratio, [(A)/(B)], is not particularly limited and typically 500 or less, and preferably 300 or less.

### (C) Alcohol

The alcohol used in the present invention is selected from the group consisting of ethanol, propanol, isopropanol, isobutyl alcohol, t-butyl alcohol. Of these, ethanol is preferably used. In the hair cosmetic of the present invention, one kind or two or more kinds of alcohols can be mixed in combination

A mixing amount of (C) the alcohol in the hair cosmetic of the present invention to the total amount of the cosmetic is 10 to 60 mass%, and preferably 20 to 50 mass%. When a mixing amount of the lower alcohol is less than 10 mass%, the stability tends to be reduced. When a mixing amount is 60 mass% or more, the cosmetic does not spread throughout hair likely causing lack of smoothness of hair.

### (D) Water

The hair cosmetic of the present invention is an aqueous low viscous cosmetic usable by being sprayed in the form of a mist. The "aqueous cosmetic" in the present specification is defined as a cosmetic containing 40 mass% or more, and preferably 50 mass% or more, of water based on the total amount of the cosmetic.

In addition to the above essential components (A) to (D), other components typically used for cosmetic products and pharmaceutical products can be optionally added to the hair cosmetic of the present invention within a range which does not affect the effects of the present invention. Examples of such components includes moisturizers, oily components, powder components, natural polymers, synthetic polymers, thickeners, UV absorbers, surfactants (provided that cationic surfactants are excluded), sequestering agents, pH adjusting agents, skin nutrients, vitamins, antioxidants, antioxidant aids, and perfumes.

The pH of the hair cosmetic of the present invention is not particularly limited and preferably 5 to 8, and particularly when a pH is adjusted within the range of 5 to 7, the hydrophobic (water repellent) effect of the hair surface is further improved.

The hair cosmetic of the present invention is an aqueous cosmetic, but the form thereof can be oil-in-water type emulsion or a solution (solubilized) form. The hair cosmetic of the present invention can be produced by a commonly used conventional production method, and is suitable to be provided in a container accommodated for spraying in the form of a mist or a spray.

The hair cosmetic of the present invention can be used as a cosmetic which is a type of applying to hair and "rinsing off" or a cosmetic which is a type of applying to hair and "leaving on" instead of rinsing off.

A heat treatment temperature is 40°C or more and 230°C or less, preferably 60°C or more and 180°C or less, and further preferably 80°C or more and 150°C or less. At less than 40°C, the holding effect of the effects is insufficient. At more than 230°C, hair may be damaged by heat.

### Examples

Hereinafter, the present invention will be further described in detail in reference to Examples but the present invention is not limited to these Examples. Note that the mixing amount is shown in mass% unless otherwise specified.

First, the hair cosmetic of each Example was prepared by a routine method with the formulations shown in Table 1 and Table 2 below.

The cosmetic of each Example was evaluated for the stability by the criteria below, and the cosmetics in which deposition was detected were not evaluated thereafter.

The hair cosmetic (1.0 g) of each Example whose safety had been confirmed was applied to a 15 cm (0.5 g) damaged hair strand and heat treated at 150°C using a hair iron.

The hair strands subjected to the treatment using the hair cosmetic of each Example were evaluated for a sense of use and water repellency (contact angle) by the following method/criteria.

Further, each of the hair strands was washed 5 times using a 10% SLES solution shampoo, which was free of a conditioning component. Water repellency (contact angle) after washing once and after washing 5 times using such a shampoo was evaluated again.

### Evaluation method and evaluation criteria

### <Stability>

The prepared cosmetics were allowed to stand for 7 days at room temperature and observed visually for the presence or absence of deposition.
A: Stable (no deposition was observed)
D: Unstable (deposition was observed)

### <Sense of use (a silky feel of hair)>

Trained researchers touched the hair strands treated with the hair cosmetic of each Example with hands and evaluate by the following evaluation criteria.

### (Evaluation criteria)

A: Hair felt very silky.
B: Hair felt silky.
C: Hair felt silky and coarseness and stickiness.
D: Hair did not feel silky.

### <Effect-holding property>

The evaluation on the sense of use (silky feel) described above was carried out 1 day later and 5 days later from the hair treatment and evaluated by the following criteria.
A: Evaluation result did not go down even when measured 5 days later.
B: Evaluation result did not go down when measured 1 day later but went down when measured 5 days later.
D: Evaluation result came down when measured 1 day later.

The "evaluation result comes down" herein means that grade A comes down to grade B, grade B comes down to grade C.

### <Water repellency (contact angle)>

Two µl of water was added dropwise to the surface of the hair strand, and the water drops were photographed under a microscope. From the photographed images, left and right angles of water drops on hair were measured, and an average value thereof was defined as a single measurement value. The same measurement was repeated 3 times, and an average value thereof was defined as a value of contact angle.

**[Table 1]**

| | **Example 1** | **Example 2** | **Example 3** | **Example 4** | **Example 5** |
|---|---|---|---|---|---|
| **(A) Crosslinked silicone POA copolymer*1** | **1** | **0.1** | **3** | **1** | **1** |
| **(B) Stearyltrimethylammonium chloride** | **0.125** | **0.125** | **0.125** | **0.05** | **0.5** |
| **(C) Ethanol** | **30** | **30** | **30** | **30** | **30** |
| **(D) Ion-exchanged water** | **Balance** | **Balance** | **Balance** | **Balance** | **Balance** |
| **Dipropylene glycol** | **5** | **5** | **5** | **5** | **5** |
| **Sodium lactate** | **0.05** | **0.05** | **0.05** | **0.05** | **0.05** |
| **Perfume** | **0.3** | **0.3** | **0.3** | **0.3** | **0.3** |
| **Total** | **100** | **100** | **100** | **100** | **100** |
| **Mixing amount ratio [(A)/(B)]** | **8.0** | **0.8** | **24.0** | **20.0** | **2.0** |
| **pH** | **5.75** | **5.88** | **5.80** | **5.69** | **5.86** |
| | | | | | |
| **Stability** | **A** | **A** | **A** | **A** | **A** |
| **Sense of use (silky feel of hair): immediately after treatment** | **A** | **A** | **B** | **B** | **A** |
| **Effect-holding property** | **A** | **B** | **A** | **A** | **A** |
| **Contact angle (before washing)** | **123.7** | **121.0** | **122.8** | **118.8** | **125.2** |
| **Contact angle (after washing once)** | **114.7** | **115.2** | **117.8** | **119.7** | **119.2** |
| **Contact angle (after washing 5 times)** | **114.5** | **99.5** | **117.3** | **116.0** | **116.8** |

| | | | | | |
|---|---|---|---|---|---|
| *1: Polysilicone-29 | | | | | |

**[Table 2]**

| | **Comparative Example 1** | **Comparative Example 2** | **Comparative Example 3** | **Comparative Example 4** |
|---|---|---|---|---|
| **(A) Crosslinked silicone POA copolymer*1** | **-** | **1** | **1** | **-** |
| **(B) Stearyltrimethylammonium chloride** | **0.125** | **-** | **0.125** | **-** |
| **(C) Ethanol** | **30** | **30** | **-** | **-** |
| **(D) Ion-exchanged water** | **Balance** | **Balance** | **Balance** | **100** |
| **Dipropylene glycol** | **5** | **5** | **5** | **-** |
| **Sodium lactate** | **0.05** | **0.05** | **0.05** | **-** |
| **Perfume** | **0.3** | **0.3** | **-** | **-** |
| **Total** | **100** | **100** | **100** | **100** |
| **Mixing amount ratio [(A)/(B)]** | **0** | **-** | **8.0** | **-** |
| **pH** | **6.85** | **5.68** | **5.22** | **-** |
| | | | | |
| **Stability** | **A** | **D** | **D** | **-** |
| **Sense of use (silky feel of hair): immediately after treatment** | **A** | **-** | **-** | **-** |
| **Effect-holding property** | **D** | **-** | **-** | **-** |
| **Contact angle (before washing)** | **117.7** | **-** | **-** | **85.5** |
| **Contact angle (after washing once)** | **88.3** | **-** | **-** | **86.2** |
| **Contact angle (after washing 5 times)** | **-** | **-** | **-** | **87.0** |

| | | | | |
|---|---|---|---|---|
| *1: Polysilicone-29 | | | | |

As shown in Table 1, the hair cosmetics of Examples 1 to 5, in which (A) the crosslinked silicone POA copolymer and (B) the cationic surfactant were combined and mixed to obtain cosmetics in the form of a mist containing (C) the lower alcohol and (D) water, were good in an effect to impart a silky feel to hair and the effect was held for an extended period of time. Further, the contact angle (water repellency) was well maintained even after washing 5 times with the shampoo.

On the other hand, in the results shown in Table 2, Comparative Example 1 in which (A) the crosslinked silicone POA copolymer was not mixed failed to hold a sense of use (a silky feel) and water repellency and had the contact angle (water repellency) equivalent to the untreated hair (Comparative Example 4) when washing once. Further, Comparative Examples 2 and 3, in which (B) the cationic surfactant or (C) the lower alcohol was not mixed, failed to prepare stable formulations.

Hereinbelow, another formulation example of the hair cosmetic of the present invention is shown.

### <Formulation Example 1>

| (Mixing components) | (mass%) |
|---|---|
| (1) Purified water | Balance |
| (2) EDTA2Na2H₂0 | 0.01 |
| (3) Stearyltrimethylammonium chloride | 0.125 |
| (4) Ethanol | 25 |
| (5) Dipropylene glycol | q.s. |
| (6) Crosslinked silicone POA copolymer (*1) | 0.25 to 1.55 |
| (7) Polyoxyethylene·polyoxypropylene-2-decyltetradecyl ether (24EO)(13PO) | q.s. |
| (8) Perfume | q.s. |
| Total | 100 |

| | |
|---|---|
| *1: Polysilicone-29 | |

## Claims

1. A hair cosmetic comprising:
(A) 0.05 to 5.0% by mass of polysilicone-29;
(B) 0.01 to 1.0% by mass of a cationic surfactant;
(C) 10 to 60% by mass of at least one alcohol selected from the group consisting of ethanol, propanol, isopropanol, isobutyl alcohol and t-butyl alcohol; and
(D) water,
wherein the cationic surfactant is a quaternary ammonium salt represented by the following formula (I): 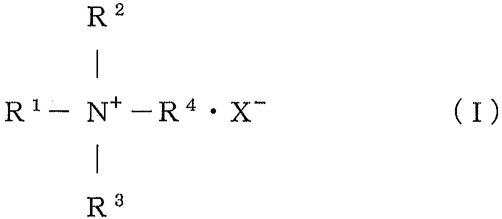
wherein R¹ is a straight chain or branched chain alkyl group having 6 to 35 carbon atoms, R², R³ and R⁴ may be the same or different and are a hydrogen atom or a straight chain or branched chain alkyl group having 1 to 3 carbon atoms, and X is a halogen ion or an organic anion, and
wherein a ratio of a mixing amount of said (A) polysilicone-29 to a mixing amount of said (B) cationic surfactant, [(A)/(B)], is 0.5 or more.

2. The hair cosmetic according to claim 1, wherein the hair cosmetic has pH of 5 to 7.

3. The hair cosmetic according to claim 1 or 2, wherein a ratio of a mixing amount of said (A) polysilicone-29 to a mixing amount of said (B) cationic surfactant, [(A)/(B)], is 0.8 or more.

4. The hair cosmetic according to any one of claims 1 to 3, wherein said hair cosmetic is used by being sprayed in the form of a mist.

5. A hair treatment method comprising applying the hair cosmetic according to any one of claims 1 to 4 to hair and performing heat treatment at 40°C to 230°C.

## Patentansprüche

1. Haarkosmetikum, umfassend:
(A) 0,05 bis 5,0 Masse-% Polysilicon-29;
(B) 0,01 bis 1,0 Masse-% eines kationischen Tensids;
(C) 10 bis 60 Masse-% mindestens eines Alkohols, ausgewählt aus der Gruppe bestehend aus Ethanol, Propanol, Isopropanol, Isobutylalkohol und t-Butylalkohol; und
(D) Wasser,
wobei das kationische Tensid ein quartäres Ammoniumsalz ist, das durch die folgende Formel (I) dargestellt wird: 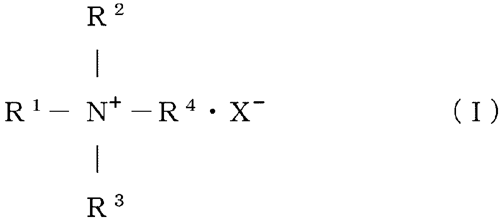
worin R¹ eine geradkettige oder verzweigtkettige Alkylgruppe mit 6 bis 35 Kohlenstoffatomen ist, R², R³ und R⁴ gleich oder verschieden sein können und ein Wasserstoffatom oder eine geradkettige oder verzweigtkettige Alkylgruppe mit 1 bis 3 Kohlenstoffatomen sind, und X ein Halogenion oder ein organisches Anion ist, und wobei das Verhältnis der Mischungsmenge des (A) Polysilicons-29 zu der Mischungsmenge des (B) kationischen Tensids, [(A)/(B)], 0,5 oder mehr beträgt.

2. Haarkosmetikum nach Anspruch 1, wobei das Haarkosmetikum einen pH-Wert von 5 bis 7 aufweist.

3. Haarkosmetikum nach Anspruch 1 oder 2, wobei das Verhältnis der Mischungsmenge des (A) Polysilicons-29 zu der Mischungsmenge des (B) kationischen Tensids, [(A)/(B)], 0,8 oder mehr beträgt.

4. Haarkosmetikum nach einem der Ansprüche 1 bis 3, wobei das Haarkosmetikum durch Sprühen in Form eines Nebels verwendet wird.

5. Haarbehandlungsverfahren, umfassend das Auftragen des Haarkosmetikums nach einem der Ansprüche 1 bis 4 auf das Haar und die Durchführung einer Wärmebehandlung bei 40°C bis 230°C.

## Revendications

1. Cosmétique capillaire comportant :
(A) 0,05 à 5,0 % en masse de polysilicone-29 ;
(B) 0,01 à 1,0 % en masse d'un tensioactif cationique ;
(C) 10 à 60 % en masse d'au moins un alcool choisi parmi le groupe constitué d'éthanol, de propanol, d'isopropanol, d'alcool isobutylique et d'alcool t-butylique ; et
(D) de l'eau,
dans lequel le tensioactif cationique est un sel d'ammonium quaternaire représenté par la formule (I) suivante : 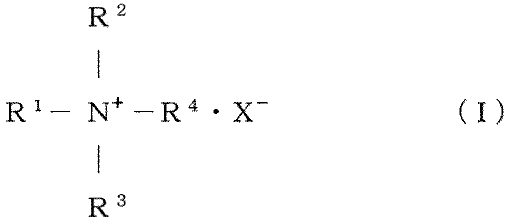
dans lequel R¹ est un groupe alkyle à chaîne droite ou à chaîne ramifiée possédant 6 à 35 atomes de carbone, R², R³ et R⁴ peuvent être identiques ou différents et sont un atome d'hydrogène ou un groupe alkyle à chaîne droite ou à chaîne ramifiée possédant 1 à 3 atomes de carbone, et X est un ion d'halogène ou un anion organique, et
dans lequel un rapport d'une quantité de mélange de ladite (A) polysilicone-29 sur une quantité de mélange dudit (B) tensioactif cationique, [(A)/(B)], est égal à 0,5 ou plus.

2. Cosmétique capillaire selon la revendication 1, dans lequel le cosmétique capillaire a un pH de 5 à 7.

3. Cosmétique capillaire selon la revendication 1 ou 2, dans lequel un rapport d'une quantité de mélange de ladite (A) polysilicone-29 sur une quantité de mélange dudit (B) tensioactif cationique, [(A)/(B)], est égal à 0,8 ou plus.

4. Cosmétique capillaire selon l'une quelconque des revendications 1 à 3, dans lequel ledit cosmétique capillaire est utilisé en étant pulvérisé sous la forme d'une brouillard.

5. Procédé de traitement capillaire comportant l'application du cosmétique capillaire selon l'une quelconque des revendications 1 à 4 sur des cheveux et la réalisation d'un traitement thermique à 40 °C à 230 °C.
